# EUROPÄISCHE PATENTANMELDUNG

(11) **EP 0 952 447 A1**
(43) Veröffentlichungstag der Anmeldung: **27.10.1999**
(21) Anmeldenummer: 98107464.4
(22) Anmeldetag: 23.04.1998
(51) Int. Cl.: G01N 27/12

(54) **Gassensor und Verwendung als Methan/Propansensor**

(71) Anmelder: SIEMENS AKTIENGESELLSCHAFT, 80333 München (DE)
(72) Erfinder: Flingelli, Gabriele, 81739 München (DE); Fleischer, Maximilian, Dr., 85635 Höhenkirchen (DE); Meixner, Hans, Prof. Dr., 85540 Haar (DE)

(57) **Zusammenfassung**

Mit einem Mehrschichtaufbau eines halbleitenden Metalloxidgassensors werden störende Gase durch den Kontakt mit beheizten Teilen des Sensors, die nicht zur gassensitiven Schicht (2) gehören, thermisch zersetzt oder durch entsprechende Katalysatorelemente (11) oder eine Katalysatorschicht (8) katalytisch umgewandelt. Der im wesentlichen für die Methandetektion gedachte Sensor weist eine erheblich verbesserte Gasselektivität auf, ist langzeitstabil, energiesparend und kann einfach hergestellt werden.

## Beschreibung

Die Erfindung betrifft einen Gassensor, der eine aus Haibleitermaterial bestehende gassensitive Schicht aufweist, die beheizt wird, um optimale Detektionseigenschaften zu erhalten.

Die Gassensoren auf der Basis von halbleitenden Metalloxidfilmen sind dadurch gekennzeichnet, daß die Sensitivität nicht selektiv auf ein bestimmtes Gas beschränkt ist. Es erzeugen grundsätzlich alle Gase mit einer bestimmten Eigenschaft, wie beispielseweise eine reduzierende Wirkung, eine Veränderung der elektrischen Leitfähigkeit und damit ein Sensorsignal. Dabei zeigen sich in der Regel bei reaktiven Gasen stärkere Sensorsignale als bei weniger reaktiven Gasen. Von schwerwiegender Bedeutung ist beispielsweise die Höhe der Reaktivität von Ethanol im Vergleich zu Methan. Dieser Sachverhalt behindert bislang die Selektion von Methan vor einem ethanolhaltigen Hintergrund.

Die Sensitität der genannten Gassensoren hängt u.a. von der Betriebstemperatur ab, wobei sich vergleichsweise hohe Temperaturen günstig zur Unterdrückung einer Feuchte- Querempfindlichkeit einsetzen lassen. Beheizte Gassensoren sind gleichzeitig unabhängig von Schwankungen der Umgebungstemperatur. Eine für diese günstigen Temperaturen hohe Leistungsaufnahme der Senorheizung steht jedoch einem wirtschaftlichen Betrieb dieser Gassensoren entgegen. Darüber hinaus führen bestimmte Gaskomponenten zu einer Vergiftung und damit zu einer verringerten Langzeitstabilität der Sensoren. Eine zusäztliche Beeinträchtigung der Langzeitstabilität ist darüber hinaus darin zu sehen, daß Sensormaterialien selbst oder Komponenten des Sensormaterials bei Betriebstemperaturen flüchtig sein können. Negative Folgen sind dann beispielsweise ein Driften des Sensorgrundwiderstandes und Veränderungen bis hin zum Verlust der Gassensitivität.

Im Stand der Technik werden nahezu für jede Gasart speziell entwickelte Sensoren eingesetzt. Auf dem Gebiet kleiner und kostengünstiger Sensoren sind elektrochemische Zellen, gassensitive Feldeffekt- Transistoren, kaloriemetrische Detektoren, Oberflächenwellen(OFW)- Chemosensoren und resistive Gassensoren auf der Basis halbleitender Metalloxide im Einsatz. Die halbleitenden Metallioxide zeichnen sich dadurch aus, daß die elektrische Leitfähigkeit des Halbleiters bei erhöhter Temperatur eine eindeutige Funktion des Partialdruckes des zu messenden Gases ist. Zur Steigerung der Selektivität sind verschiedene Entwicklungen bekannt. Beispielsweise wird die gassensitive Schicht dotiert, die Sensoroberfläche wird modifiziert oder es werden Sensor-Arrays oder spezielle Auswerteverfahren eingesetzt. Darüber hinaus können Gasfilter oder andere Verfahren zur Gasvorbehandlung angewandt werden. Zur Verbesserung, d.h. zur Minimierung der obengenannten hohen Leistungsaufnahine läßt sich bisher eine Miniaturisierung des Sensors anführen.

In der internationalen Anmeldung PCT/DE 96/00833 wird eine Gassensoranordnung beschrieben, mittels der Querempfindlichkeiten an einem Gassensor auf störende Gase reduziert werden können. Dazu ist dem Gassensor ein räumlich getrenntes Katalysatorfilter vorgeschaltet. In oder an diesem Katalysatorfilter werden störende Gase in nicht störende Komponenten umgewandelt. Das Katalysatorfilter wird durch eine eigene Heizung beheizt.

Der Erfindung liegt die Erfindung zugrunde,einen Gassensor der oben beschriebenen Art mit verbesserter Sensitivität und optimiertem Leistungsverbrauch bereitzustellen, der darüber hinaus eine hohe Langzeitstabilität aufweist.

Die Lösung dieser Aufgabe geschieht durch die Merkmalskombinationen im Anspruch 1, im Anspruch 4 oder im Anspruch 5.

Vorteilhafte Ausgestaltungen können den Unteransprüchen entnommen werden.

Der Erfindung liegt der Erkenntnis zugrunde, daß die Selektivität von halbleitenden Metalloxid-Gassensoren durch bestimmte Maßnahmen wesentlich gesteigert werden kann. Querempfindlichkeiten können fast vollständig unterdrückt werden. Die bestimmten Maßnahmen bestehen darin, daß ein auf einem Substrat befindliches gassensitives Element, das mit dem Substrat zusammen durch eine Heizungsstruktur beheizt wird zunächst einmal unter Bildung eines über der gassensitiven Schicht befindlichen Innenraumes mit einer Deckplatte überdeckt bzw. abgedeckelt wird. Somit entsteht ein Mehrschichtaufbau, wobei die Deckplatte in einem bestimmten Abstand von dem Substrat bzw. der gassensitiven Schicht plaziert ist. Der Gasaustausch mit der zu messenden Atmosphäre wird jeweils gewährleistet. Die somit entstandene Gassensorvorrichtung wird durch die einzige Heizungsstruktur erhitzt, so daß die Deckplatte ungefähr die gleiche Temperatur wie die gassensitive Schicht aufweist. Somit gelingt eine Erwärmung des Gasvolumens verbunden mit einer konvektiven Gaszuführung , wobei der thermische Zerfall weniger stabiler Gase induziert wird, bevor sie zur gassensitiven Schicht gelangen. Somit erreichen nur stabile Komponenten die eigentliche Sensoroberfläche.Damit wird eine erhöhte Selektivität erzielt. Darüber hinaus bietet diese Vorrichtung durch die Abdeckelung eine thermische Isolierung, die die Leistungsaufnahme der Sensorheizung verringert. Zusätzlich wird eine Abreaktion und Ablagerung von Sensorgiften an den Wänden des Mehrschichtaufbaues bereits in den Zonen vor dem Sensormaterial ermöglicht. Eine Erhöhung der Langzeitstabilität ergibt sich auch daraus, daß die Verflüchtigung von Sensormaterial oder von Komponenten des Sensormaterials verringert wird. Dies geschieht dadurch, daß sich im Mehrschichtaufbau im Vergleich zu einer herkömmlichen Bauweise eines Gassensors zwischen dem Festkörper und der Gasphase ein bestimmter Konzentrationsgradient einstellen kann, wodurch der Stofftransport vom Sensormaterial in die Gasphase verringert wird.

Werden zusätzlich zur Deckplatte beispielsweise katalytisch wirkende Materialien angeboten, so kann zusätzlich zum thermischen Zerfall von störenden Gasen auch eine katalytische Gasvorbehandlung stattfinden. Besteht der Katalysator aus dem gleichen Material wie die gassensitive Schicht des Sensors, so weisen beide Materialien, die durch die Heizung auch auf ungefähr gleiche Temperatur gebracht werden, die gleiche Reaktivität gegenüber Gasen auf. Gase, deren Sensorreaktion auf Redoxreaktionen zurückzuführen sind, rufen in der Regel dann ein starkes Sensorsignal hervor, wenn sie leicht zu oxidieren sind. Eine Detektion solcher Gase bereitet in der Regel keine Probleme, solange die Sensorsignale der Störgase unterhalb einer bestimmten Schwelle liegen, also nur schwache Wechselwirkungen mit der gassensitiven Schicht aufweisen. Im umgekehrten Fall ist die Detektion eines Gases mit schwacher Wechselwirkung am Sensor jedoch nicht möglich, solange ein Gas mit starker Wechselwirkung anwesend ist (Störgas). Mit der Erfindung wird gerade diese Kenntnis der Reaktionen zwischen Gas und gassensitivem Material ausgenutzt. Gase, die als Störgas unerwünscht sind, da sie ein starkes Störsignal hervorrufen, können bereits thermisch oder katalytisch, an einem Katalysator aus dem gleichen Material wie der Sensor, abreagieren. Wechselwirkungen von Störgasen an der gassensitiven Schicht werden dadurch weitestgehend eliminiert.

Eine Überdeckung der gassensitiven Schicht mit einem Katalysator aus einem porösen Material, das dasselbe Material ist, aus dem die gassensitive Schicht besteht, liefert durch die Vielzahl der Poren eine Ausführungsvariante mit vielen kleinen Hohlräumen. Damit wird die thermische und katalytische Vorbehandlung vielfach kombiniert.

Im folgenden werden anhand von schematischen Figuren weitere Ausführungsbeispiele beschrieben:
Figur 1 zeigt den herkömmlichen Aufbau eines Halbleiter gassensors,
Figur 2 zeigt einen Mehrschichtaufbau eines Gassensors,
Figur 3 zeigt einen Querschnitt der Darstellung entsprechend Figur 2,
Figur 4 zeigt einen Mehrschichtaufbau eines Gassensors mit geschlossenem Innenraum und mit über Diffusion gesteuer tem Gasaustausch,
Figur 5 zeigt ein Diagramm einer Sensormessung an Gasen dem Sensorsignale für den Vergleich zwischen einem Mehr schichtaufbau und einem herkömmlichen Aufbau eines Gassensors zu entnehmen sind,
Figur 6 zeigt einen geschlossenen Innenraum über der gassensitiven Schicht, wobei die Gaszuführ durch Katalysator geschieht,
Figur 7 zeigt einen Gassensor mit einer direkt über der gassensitiven Schicht aufgebrachten Katalysatorschicht, wobei eine elektrisch isolierende Zwischenschicht einge bracht ist,
Figur 8 zeigt die Abhängigkeit der gesamten Sensorempfind lichkeit von der Dicke der Zwischenschicht bei einem Sensor nach Figur 7,
Figur 9 zeigt den Vergleich zwischen einem Galliumoxidsensor, der einmal mit und einmal ohne einer GalliumoxidKatalysatorschicht betrieben wird (Sensor nach Figur 7),
Figur 10 zeigt den Vergleich eines Galliumoxidssensors, der einmal mit und einmal ohne einen Galiumoxidkatalysator betrieben wird (Sensor nach Figur 6).

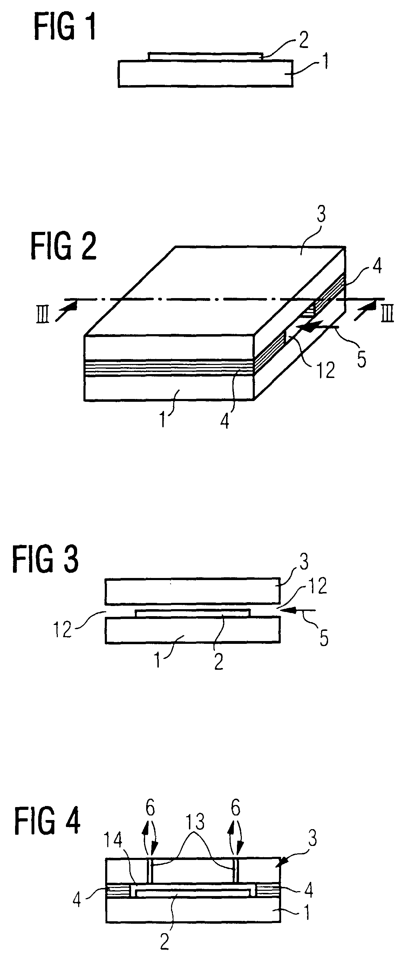

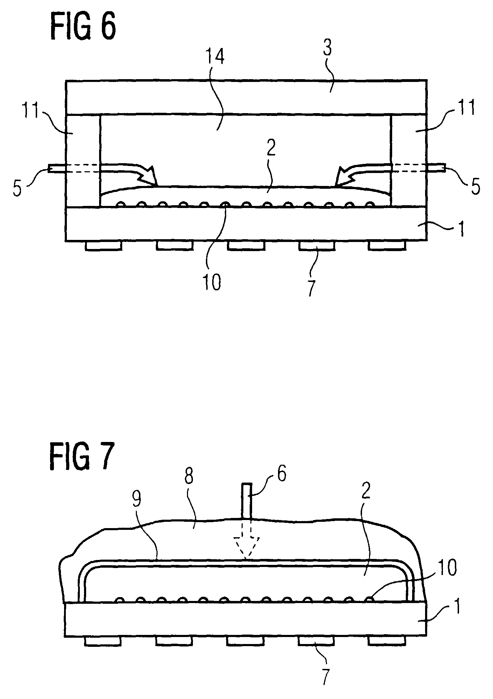

Wie bereits erwähnt sind halbleitende Metalloxid-Gassensoren in der Lage in Gegenwart von reduzierend oder oxidierend wirkenden Gasen ihre Leitfähigkeit zu ändern. Dabei ist eine Wechselwirkung der Gasmoleküle an oder mit der Sensoroberfläche entscheidend. In der Natur dieser Wechselwirkungen (Elektronen-Donor-Akzeptor-Wechselwirkungen, Redox-Reaktionen) liegt es begründet, daß unabhängig vom verwendeten Sensormaterial eine Querempfindlichkeit der Sensoren auftritt. So gelingt beispielsweise der Nachweis von Methan vor einem ethanolhaltigen Hintergrund nur schwer, da zwar beide Gaskomponenten eine Wechselwirkung mit der Sensoroberfläche bewirken, aber die reduzierende Wirkung des Ethanols stärker zum Tragen kommt als die des Methans. Die Erfindung stellt eine wesentliche Weiterentwicklung eines in Figur 1 dargestellten lediglich aus einem Substrat 1 und einer darauf aufgebrachten halbleitenden gassensitiven Schicht 2 bestehenden Gassensors dar. Der erfindungsgemäße Mehrschichtaufbau der in verschiedenen Varianten den Figuren 2 bis 7 zu entnehmen ist, weist folgende Merkmale verbunden mit entsprechenden Vorteilen auf:
- das Sensorelement enthält eine Deckplatte 3 oder eine Katalysatorschicht 8,
- der Abstand zwischen dem plattenförmigen Substrat 1 und der Deckplatte 2 wird entsprechend der Figuren 2, 3 und 4 mit Distanzstücken, beispielsweise ausgehärtete Keramikpaste, oder funktionsgleichen Mitteln dargestellt,
- für den Austausch mit dem umgebenden Gas werden entweder entsprechend der Figuren 2 und 3 zwei Öffnungen angeboten, so daß der Gasaustausch durch Konvektion geschieht oder eine oder mehrere Öffnungen 13 erlauben einen Gasaustausch entsprechend Figur 4 durch Diffusion,
- der Weg des Gases von den Öffnungen 12, 13 zur gassensitiven Schicht 2 ist somit ausreichend lang, daß eine vollständige Abreaktion der instabilen Gaskomponenten bewirkt wird. Ohne einen Katalysator beruht diese Abreaktion lediglich auf thermischem Zerfall. Werden entsprechend der Figuren 6 und 7 Katalysatorelemente 11 oder eine Katalysatorschicht 8 eingesetzt, so liegt eine katalytische Gasvorbehandlung unter Umständen kombiniert mit einer thermischen Gasbehandlung vor.

Erfindungsgemäß ausgeführte Gassensoren weisen eine ganze Reihe von Vorteilen auf:
- die Gassensoren sind in einfachen Prozeßschritten kostengünstig herstellbar,
- die für die Reaktionen am Katalysator notwendige Wärme wird durch die einzige am Sensor vorhandene Heizung erzeugt,
- zwischen Katalysatorelementen 11 bzw. Katalysatorschicht 8 und sensitiver Schicht 2 treten keine Wechselwirkungen auf, so daß eine Veränderung des sensitiven Materials durch Reaktion mit dem Katalysatormaterial ausgeschlossen ist. Es entstehen keine Kurzschlüsse der Sensorschicht, keinerlei Dotierung des Sensors und das Sensormaterial zersetzt sich nicht durch chemische Reaktionen,
- der Katalysator wirkt selektiv, da er störende Gase bzw. Komponenten abbaut, aber die relevanten Gase nicht angreift,
- ein mit einem Katalysator versehener Sensor ist somit langzeitstabil, weil eine Vergiftung des Sensormaterials unterbleibt.

Somit sind erfindungsgemäße Gassenoren wesentlich selektiver, langzeitstabiler, und bezüglich der Heizleistung sparsamer als bisher bekannte Sensoren. Darüber hinaus sind sie kostengünstig herstellbar.

Die thermische Isolierung der Gassensoren wird durch die zusätzliche Aufbringung einer weiteren Schicht in Form einer Deckplatte 3 oder in Form der Katalysatorschicht 8 bewirkt, wie es in den Figuren 2 bis 7 dargestellt ist. Gegenüber einem herkömmlichen Sensoraufbau wird somit ein Wärmetransport nach außen vermindert, obwohl diese zusätzliche Schicht mit beheizt werden muß. Insgesamt wird die Leistungsaufnahme der Sensorheizung verringert.

Sensorgifte können bereits auf dem Weg zur gassensitiven Schicht abgelagert werden. Durch einen bei entsprechender Anordnung der Sensorheizung unter der sensitiven Schicht 2 bedingten Temperaturgradienten wird zudem ein Reinigungseffekt erzielt. In Verbindung damit wird mit einer Anordnung ensprechend der Figur 4 durch die Einstellung eines Konzentrations-gradienten die Abreicherung von Sensormaterial verringert.

Dies gilt in eingeschränkter Form auch für die Beispiele entsprechend der Figuren 2, 3, 6 und 7.

Als Anwendungsfälle sind beispielsweise methanselektive Sensoren für Warngeräte in Haushalten mit Erdgasversorgung, in rergwerken usw. vorgesehen. Allgemein sind darüber hinaus Sensoren für die Detektion von umwelt- oder gesundheitsrelevanten Gasen, sowie für die Prozeßüberwachung und - steuerung zu nennen. Dabei ist von Bedeutung, daß diejenigen Gase, auf die die verwendeten Sensoren Querempfindlichkeiten aufweisen, einerseits leichter zerfallen als die zu detektierende Gaskomponente und daß die Zerfallsprodukte am Sensor keinen Effekt hervorrufen. Im Falle eines Methanwarngerätes für Haushalte stellen bisher Alkoholdämpfe aus Reinigern oder Kochdünste ein gravierendes Problem dar, insbesondere wegen der hohen Sensitivität vieler Metalloxide auf Ethanol. Sowohl Ethanol, als auch Aceton und andere Lösungsmittel zeigen an Metalloxidgassensoren starke Sensorsignale, die einerseits die Detektion von möglicherweise anwesendem Methan verhindern und andererseits selbst als Fehlalarm unerwünscht sind. Mit einem Sensor nach der neuen Bauweise kann der Zerfall des Ethanols als weniger stabile Komponente zu Kohlendioxid und Wasser bewirkt werden, wobei diese Reaktionsprodukte an den meisten Sensoren keine Reaktion zeigen. Experimente an Galliumoxidsensoren beweisen diese Zusammenhänge.

Werden beispielsweise Sensoren betrachtet, deren gassensitives Material selbst oder Komponenten davon bei den für ausreichendes Ansprechverhalten des Sensors nötigen hohen Temperaturen einen erheblichen Dampfdruck besitzen, so können durch erfindungsgemäße Gassensoren bestimmte Nachteile ausgeräumt werden. Als Beispiel ist ein Sensor aus Aluminium-Vanadat zu nennen. Dessen Sensitivität auf Stickoxid wird vermutlich durch einen geringen Überschuß an Vanadium-(V)-Oxid verursacht. Diese Komponente besitzt bei einem Schmelzpunkt von 680°C und einer Sensoebetriebstemperatur von 500°C einen erheblichen Dampfdruck, ist also relativ leicht flüchtig. Die Abreicherung dieser Komponente wird durch den erfindungsgemäßen Mehrschichtaufbau verringert.

Es wird nochmals darauf hingewiesen, daß der erfindungsgemäße Mehrschichtaufbau trotzdem einen einfachen Gassensor erbringt, der kostengünstig herzustellen ist. Die Summe seiner Vorteile stehen in keinem Vergleich zum Aufwand. Für die Anwendung des neuen Mehrschichtaufbaues wird zunächst an die Verbesserung der derzeit bestehenden Gassensoren gedacht. Als Material für eine Deckplatte 3 und evtl. für die Abstandselemente 4 kommen temperaturstabile Keramiken in Betracht, wie sie auch als Substrat 1 verwendet werden. Als Verbindungsmethoden können beispielsweise 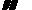 grüne Keramik", die sich durch einen Temperschritt mit ihrer Unterlage verbindet und aushärtet oder Keramikkleber oder auch Glaspaste verwendet werden, so daß eine sichere Verbindung der Elemente gewährleistet ist.

Eine in den Figuren nicht dargestellte Möglichkeit von einem Sensor mit reiner thermischer Zersetzung von störenden Gasen zu einem Sensor mit kombiniertem thermischen und katalytischen Zerfall zu gelangen, besteht darin, die Deckplatteninnenseite mit katalytisch aktiver Substanz zu überziehen. Dies wird durch einen einfachen Verfahrensschritt erreicht. Eine weitere Anwendungsvariante besteht im Aufbau eines SensorArrays mit einem Sensor nach der erfindungsgemäßen Mehrschichtbauweise und einem herkömmlich aufgebauten Sensor. Dies erlaubt eine Aufschlüsselung durch die unterschiedlich erhaltenen Sensorsignale, so daß im Falle des Methanwarngerätes für Haushalte nach tatsächlich gefährlichen Konzentrationen an Methan und nach sonstigen Luftbelastungen unterschieden werden kann.

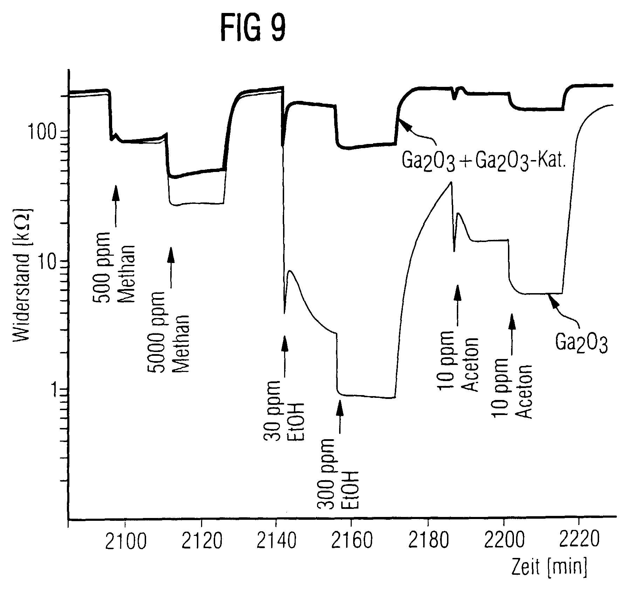

Die Figur 7 zeigt die Realisierung einer Variante der Erfindung, wobei ein Galliumoxidsensorelement mit einer porösen Galliumoxidschicht aus getemperter Galliumoxid-Keramikpaste überzogen ist. Zwischen den Schichten 2 und 8 ist eine gasdurchlässige Zwischenschicht 9 aus nichtleitendem Siliziumdioxid vorhanden, die eine elektrische Isolierung der beiden Galliumoxidschichten darstellt. Die Gassensitivität solcher mit Katalysatorelementen kombinierten Sensoren auf Ethanol und Aceton ist, wie in Figur 9 dargestellt, maßgeblich gesunken. Allerdings tritt auch eine leichte Verminderung der Gesamtempfindlichkeit auf, die auf die Dicke bzw. Materialstärke der jeweiligen durch Abscheidung von Hexamethyldisiloxan (HMDS) gewonnenen SiO₂-Zwischenschicht zurückzuführen ist. Eine andere Plazierung des Katalysators zeigt Figur 6. Darin wird der prinzipielle Aufbau der Figuren 2 bis 4 zugrundegelegt, wobei innerhalb des Mehrschichtaufbaues ein die gassensitive Schicht 2 beinhaltender Innenraum 14 dargestellt wird. In figur 6 sind die Gaseintrittsöffnungen 12,13, d.h. die Katalysatorelemente 11 aus porösem Ga₂O₃, aus getemperter Ga₂O₃-Keramikpaste. Mit einem nach Figur 6 ausgestalteten Sensor wurden die beschriebenen Vorteile erzielt.

Zu Figur 2 ist ergänzend anzumerken, daß die Beabstandung der Deckplatte 3 zum Substrat 1 durch Abstandselemente 4 geschieht, die derart plaziert sind, daß Öffnungen 12 dargestellt werden, so daß ein Gas konvektiv über die im nicht sichtbaren Innenraum 14 vorhandene gassensitive Schicht 2 streichen kann. Der Schnitt III-III ist in Figur 3 dargestellt. Darin ist erkennbar, daß ein entsprechend dem Gasstrom 5 strömendes Gas beim Eintritt in den Sensor an den Öffnungen 12 zunächst Kontakt mit dem beheizten Sensorgehäuse hat. Bevor die gassensitive Schicht 2 erreicht wird, werden thermisch bedingte Zerfallsreaktionen induziert.

Figur 4 zeigt eine weitere Ausgestaltung der Erfindung. Darin ist der Innenraum 14 abgeschlossen und die Gaszuführung erfolgt lediglich über Öffnungen 13 mit sehr kleinem Durchmesser, beispielsweise kleine Bohrungen. Diese Ausführungsform eines Gassensors verringert die Verflüchtigung des Sensormaterials wesentlich. Eine Verringerung der elektrischen Leistungsaufnahme für die Beheizung des Sensors kann erzielt werden, obwohl sich die äußere Oberfläche des Gassensors im Verhältnis zur Darstellung in Figur 1 vergrößert hat.

Figur 6 zeigt zusätzlich zu dem bisher Beschriebenen die Heizungsstruktur 7 an der Unterseite eines flachliegenden plattenförmigen Substrates 1 und die Widerstandsmeßstruktur, die interdigital (kammartig) ausgebildet ist und in Kontakt mit der gassensitiven Schicht 2 steht. Der Gasstrom 5 gelangt durch die Katalysatorelemente 11 in den Innenraum 14 und somit auf die gassensitive Schicht 2, wobei störende Gase thermisch und katalytisch abgebaut werden.

Figur 7 stellt die Ausführungsform eines erfindungsgemäßen Gassensors dar, die ohne Innenraum 14 ausgebildet wird. Die mittelbar oder unmittelbar auf der gassensitiven Schicht aufgebrachte Katalysatorschicht 8 ist porös und läßt somit eine Gasdiffusion 6 in Richtung auf die gassensitive Schicht 2 zu. Existiert eine Zwischenschicht 9, so ist auch diese gasdurchlässig. Die Zwischenschicht 9 dient lediglich zur elektrischen Isolierung zwischen den Schichten 2 und 8. Entfällt die Zwischenschicht 9, so ist dafür Sorge zu tragen, daß die Leitfähigkeiten der Schichten 2 und 8 sich wesentlich unterscheiden und somit ein Sensorsignal, das über die Widerstandesmeßstruktur 10 abgegriffen wird, im wesentlichen von der Schicht 2 stammt. Die Katalysatorschicht 8 sollte somit eine wesentlich kleinere Leitfähigkeit aufweisen als die gassensitive Schicht 2.

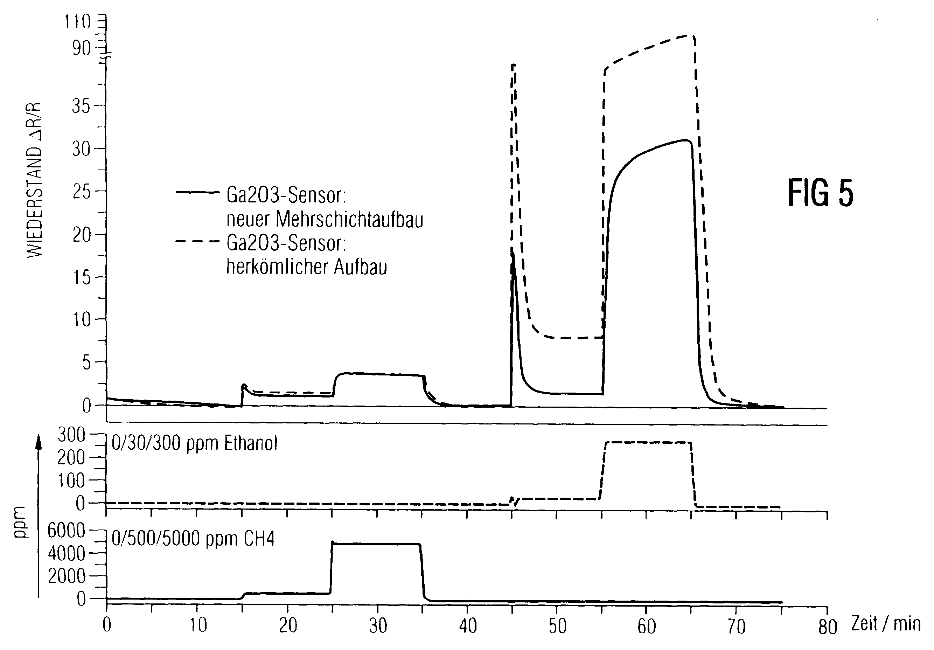

Die Figur 5 zeigt ein Meßdiagramm von Galliumoxidsensoren, die mit herkömmlichem Aufbau oder mit erfindungsgemäßem Mehrschichtaufbau hergestellt worden sind. Der auf der Ordinate aufgetragene Sensorwiderstand verändert sich bei einer Zugabe von Methan oder Ethanol. Es ist erkennbar, daß das Sensorsignal bei den zugegebenen Konzentrationen von Ethanol an einem Mehrschichtgassensor geringer ausfällt, als bei einem Sensor herkömmlicher Bauart. Es ist denkbar, einen Sensor herkömmlicher Bauart als Referenz in einem Sensorarray einzusetzen.

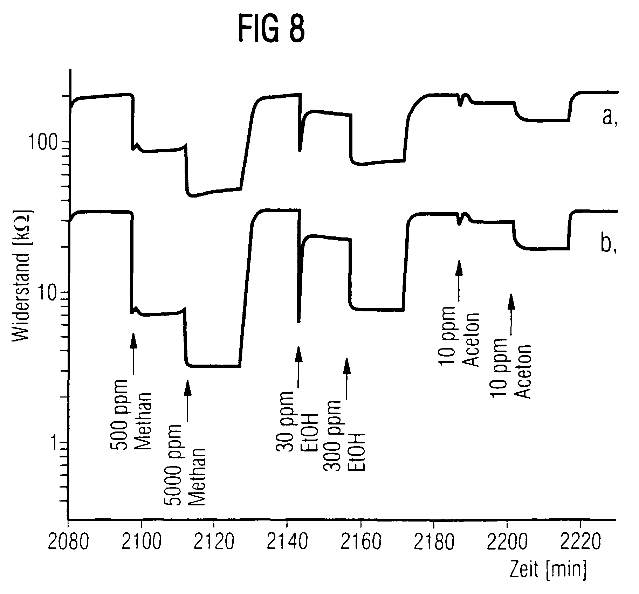

In Figur 8 sind die Kurven a) und b) dargestellt. Somit läßt sich die Abhängigkeit der gesamten Sensorempfindlichkeit von der Dicke der SiO₂-Zwischenschicht 9 aufzeigen, wobei die Kurve a) für eine größere Materialstärke der Zwischenschicht 9 steht. Die Kurve b) bedeutet eine geringere Zwischenschichtdicke. Die eingesetzten Gase sind von links nach rechts gelesen Methan in zwei verschiedenen Konzentrationen, Ethanol in zwei verschiedenen Konzentrationen und Aceton in zwei verschiedenen Konzentrationen.

Die Figur 9 zeigt Sensorsignale, die in Verbindung mit einem Sensor entsprechend Figur 7 aufgenommen worden sind. Dabei wurden die gleichen Gase mit gleichen Konzentrationen entsprechend Figur 8 eingesetzt. Die untere Kurve liefert ein herkömmlicher Galliumoxidsensor. Die obere Kurve liefert ein Galliumoxidsensor mit einer Katalysatoreinrichtung. Es ist deutlich zu erkennen, daß Aceton im Verhältnis zu Methan eine geringe Änderung des Sensorsignales verursacht. Eine Zugabe von Ethanol bewirkt eine mittlere Zunahme des Sensorsignals, die jedoch unterhalb des Ausschlages bei Methan liegt, was bei einem herkömmlichen Sensor entsprechend der unteren Kurve umgedreht ist. Der Sensor ist hierzu mit einem Gasstrom von 1 l/min synthetischer Luft mit entsprechenden Zugaben von Methan, Ethanol und Aceton betrieben worden und seine Temperatur betrug 800°C.

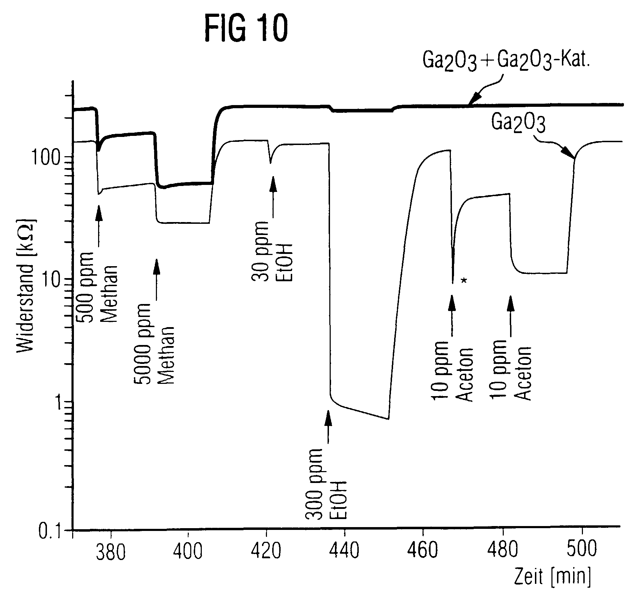

In Figur 10 ist ein Diagramm dargestellt, das mit einem Sensor entsprechend Figur 6 aufgenommen wurde, wobei der Sensor ebenfalls bei 800°C betrieben wird und der Gasstrom 1 l/min synthetische Luft mit 3% relativer Feuchte beträgt. Die untere Kurve gibt wiederum ein Sensorsignal eines herkömmlichen Galliumoxidsensors wieder. Die obere dick gezeichnete Kurve ist von einem erfindungsgemäßen, mit Katalysator versehenen Gassensor, wobei Ausschläge aufgrund von Vorhandensein von Aceton oder Ethanol fast völlig beseitigt sind. Der Sensor reagiert lediglich auf verschiedene Konzentrationen von Methan mit unterschiedlichen Ausschlägen.

Gemäß einer bevorzugten Weiterbildung der Erfindung ist vorgesehen, die erfindungsgemäße gassensitive Schicht aus Galliumoxid so auszubilden, daß sie eine Dicke von etwa 10 nm bis etwa 100 µm aufweist. Insbesondere wird die gassensitive Schicht in einer Dicke zwischen etwa 100 nm und etwa 20 µm dargestellt.

## Patentansprüche

1. Gassensor bestehend aus:
- einem Substrat (1),
- einer auf dem Substrat (1) aufgebrachten Heizungsstruktur (7),
- einer auf dem Substrat aufgebrachten gassensitiven Schicht (2) aus Halbleitermaterial,
- einer mit der gassensitiven Schicht (2) zusammenwirkenden Widerstandsmeßstruktur (10),
- einer die gassensitive Schicht (2) überdeckende Deckplatte (3), die von dem Substrat (1) durch Abstandselemente (4) unter Bildung eines Innenraumes (14) beabstandet ist, wobei durch Öffnungen (12) in der Deckplatte (3) oder in den Abstandselementen (4) Gas zur gassensitiven Schicht (2) konvektiv zuführbar ist.

2. Gassensor nach Anspruch 1,
worin die Abstandselemente (4) mit der Deckplatte (3) den Innenraum (14) über der gassensitiven Schicht (2) abschließen und mindestens eine Öffnung (13) mit kleinem Durchmesser in der Deckplatte (3) vorgesehen ist, durch die Gas in den Innenraum (14) zur gassensitiven Schicht (2) durch Diffusion zuführbar ist.

3. Gassensor nach Anspruch 1 oder 2,
worin die Innenseite der Deckplatte (3) mit katalytisch aktiver Substanz überzogen ist.

4. Gassensor bestehend aus:
- einem Substrat (1),
- einer auf dem Substrat (1) aufgebrachten Heizungsstruktur (7),
- einer auf dem Substrat (1) aufgebrachten gassensitiven Schicht (2) aus Halbleitermaterial,
- einer mit der gassensitiven Schicht (2) zusammenwirken den Widerstandsmeßstruktur (10),
- einer die gassensitive Schicht (2) überdeckende Deckplatte (3) die vom Substrat (1) unter Bildung eines abgeschlossenen Innenraums (14) beabstandet ist, wobei Gas durch aus dem gleichen Material wie die gassensitive Schicht (2) bestehende Katalysatorelemente (11) zur gassensitiven Schicht (2) zuführbar ist.

5. Gassensor bestehend aus:
- einem Substrat (1),
- einer auf dem Substrat (1) aufgebrachten Heizungsstruktur (7),
- einer auf dem Substrat aufgebrachten gassensitiven Schicht (2) aus Halbleitermaterial,
- einer mit der gassensitiven Schicht (2) zusammenwirkenden Widerstandsmeßstruktur (10),
- einer die gassensitiven Schicht (2) abdeckende gasdurchlässige Katalysatorschicht (8) aus dem gleichen Material wie die gassensitive Schicht (2),
- einer gasdurchlässigen elektrisch isolierenden Zwischenschicht (9) zwischen der gassensitiven Schicht (2) und der Katalysatorschicht (8).

6. Gassensor nach Anspruch 5,
worin die Zwischenschicht (9) aus SiO₂ besteht.

7. Gassensor nach Anspruch 6,
worin die Zwischenschicht (9) entfällt und die gasdurchlässige Katalysatorschicht (8) derart präpariert ist, dass deren elektrische Leitfähigkeit klein gegenüber der Leitfähigkeit der gassensitiven Schicht (2) ist.

8. Gassensor nach einem der vorhergehenden Ansprüche,
worin die gassensitive Schicht aus Galliumoxid (Ga₂O₃) besteht.

9. Gassensor nach einem der Ansprüche 4-7,
worin die Katalysatorelemente (11) oder die Katalysatorschicht (8) aus getemperter Galliumoxid-Keramikpaste bestehen.

10. Gassensor nach Anspruch 9,
worin die Katalysatorelemente (11) bzw. die Katalysatorschicht (8) neben der getemperten Galliumoxidpaste noch weitere katalytisch aktive Stoffe beinhaltet.

11. Gassensor nach Anspruch 10,
worin als weitere katalytisch wirkende Stoffe Pt, Rh, Pd, Au oder katalytisch aktive Oxide wie CeO₂ oder La₂O₃ verwendet werden.

12. Verwendung eines Gassensors nach einem der Ansprüche 1 bis 9 zur Detektion von Methan und/oder Propan.
